# EUROPEAN PATENT APPLICATION

(11) **EP 2 316 518 A1**
(43) Date of publication of application: **04.05.2011**
(21) Application number: 10189239.6
(22) Date of filing: 28.10.2010
(51) Int. Cl.: A61M 25/00

(54) **Medical tube and catheter using the same**

(30) Priority: 29.10.2009 JP 2009249422
(71) Applicant: Asahi Intecc Co., Ltd., Nagoya-shi, Aichi 463-0024 (JP)
(72) Inventor: Hatano, Yoshio, Nagoya-shi Aichi 463-0024 (JP); Kumai, Toshiko, Nagoya-shi Aichi 463-0024 (JP); Niwa, Shinji, Nagoya-shi Aichi 463-0024 (JP)
(74) Representative: Zimmermann, Tankred Klaus

(57) **Abstract**

Provided is a medical tube (10) that is capable of preventing thrombi from adhering to the inside of a side hole (40,50) thereof such that a required and sufficient opening area of the side hole is maintained, as well as of preventing a kink from being generated in, e.g., a catheter using the medical tube with the side hole. A side hole is provided through a side surface of a catheter body (12). A strand (26a,26b) of a braid member in the vicinity of the side hole is covered with a resinous protective resin portion, thus being kept from exposure at the inner peripheral surface of the side hole.

## Description

### Cross Reference to Related Applications

This application is based on Japanese Patent Application No. 2009-249422 filed with the Japan Patent Office on October 29, 2009, the entire content of which is hereby incorporated by reference.

### Technical Field

The present invention relates to medical tubes having a hole in a side surface thereof, and catheters using the same.

### Background Art

Conventional medical instruments, such as catheters used by being inserted into tubular organs and intracorporeal tissues, e.g., vessels, digestive tracts, and ureters include one constituted by a medical tube configured such that a metallic braid member is interposed between a resinous inner resin layer and a resinous outer resin layer. Such medical tubes are used in catheters typified by, e.g., cardiac guiding catheters. The medical tubes of this type include one provided with one or a plurality of holes (hereinafter "side holes") in a side surface of the above-mentioned medical tube for the purpose of securing a bloodstream during an operation (see, e.g., JP 07-328123A and JP 2005-531388A).

### Summary of Invention

In case of providing a side hole in the medical tube, blood passing through the side hole may adhere to the inner peripheral surface of the side hole in the form of thrombi, thus causing substantial reduction in diameter of the side hole. In such a condition, there may be a possibility that a bloodstream is not secured sufficiently while an operation is being performed with a catheter and the like.

A conceivable preventive method for this possibility is such that, for example, the number of the side hole or the diameter of the hole is increased so as to secure a little large opening area in advance. Forming a medical tube by this method however leads to reduction in structural strength of the medical tube and may consequently contribute to occurrence of a kink (yielding or crush) in, e.g., the catheter using the medical tube.

The present invention was made in view of the foregoing circumstances, and it is an object of the invention to provide a medical tube that is capable of preventing thrombi from adhering to a side hole thereof such that a required and sufficient opening area of the side hole is maintained, as well as of preventing a kink from being generated in the catheter or the like using the medical tube with the side hole.

The inventors studied further into the above object. As a result, the inventors focused on a relationship between the braid member constituting the medical tube and thrombi. More specifically, the inventors found that, in case where blood flows in the side hole with strands of the braid member exposed at the inner wall of the side hole, blood is prone to adhere in the form of thrombi to the exposed portion of the strands, and in particular, of the strands made of metal. Based on the result, the present invention provide solutions to the above object through the following features.

A medical tube according to the present invention includes: an inner resin layer including a resinous tubular body having inside a lumen extending longitudinally; a braid member including at least one strand disposed around an outer periphery of the inner resin layer; an outer resin layer covering an outer periphery of the braid member; and at least one side hole passing through the outer resin layer and the inner resin layer to communicate with the lumen. The strand of the braid member provided in a vicinity of the side hole is covered with a resinous protective resin portion.

### Brief Description of Drawings

The foregoing and other objects, features, aspects and advantages of the invention will become more apparent from the following detailed description when taken in conjunction with the accompanying drawings.
Fig. 1

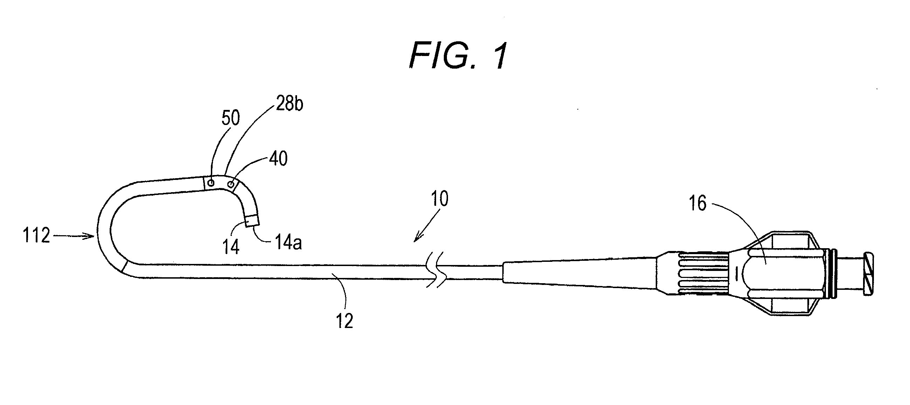

   Fig. 1 illustrates an overall view of a catheter using a medical tube of an embodiment of the invention;
Fig. 2

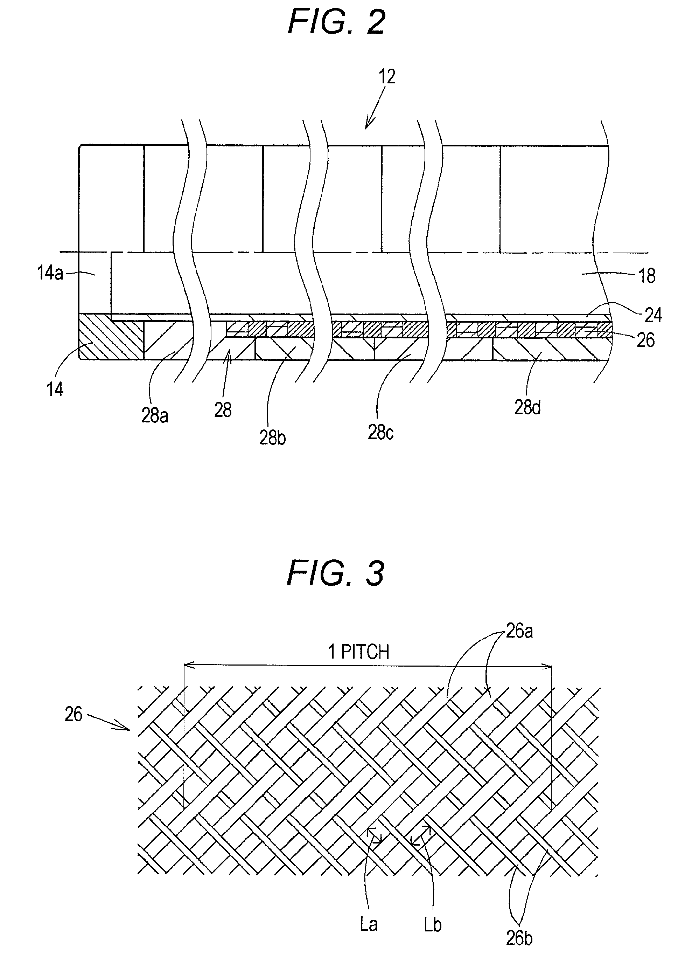

   Fig. 2 illustrates a cross-sectional view of a tip portion of the catheter of Fig. 1.
Fig. 3
   Fig. 3 illustrates a braid member.
Figs. 4A and 4B

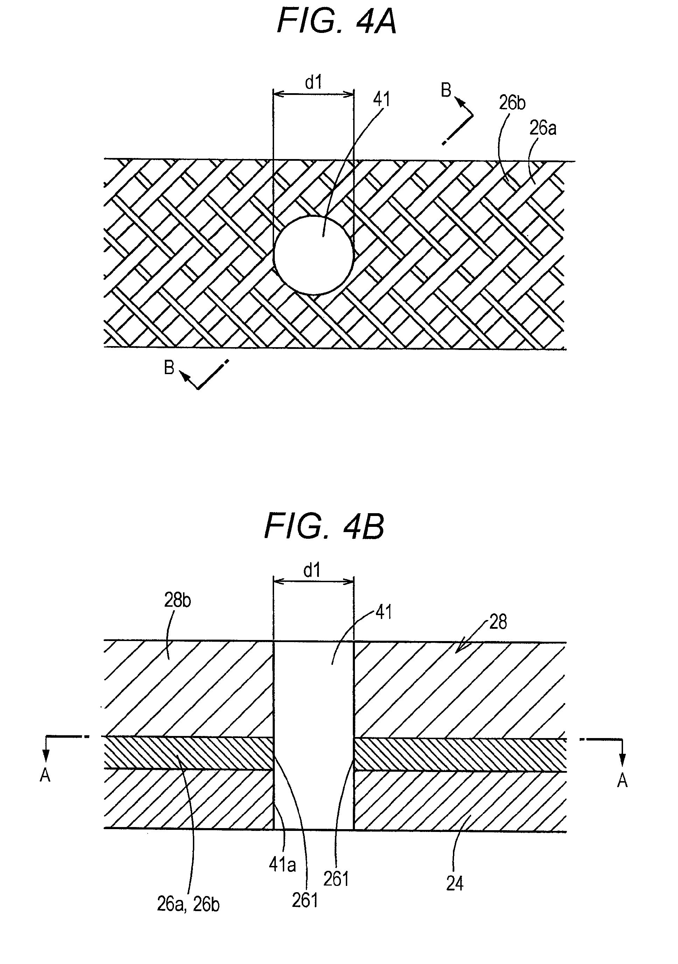

   Figs. 4A and 4B illustrate a first step for forming a side hole.
Figs. 5A and 5B

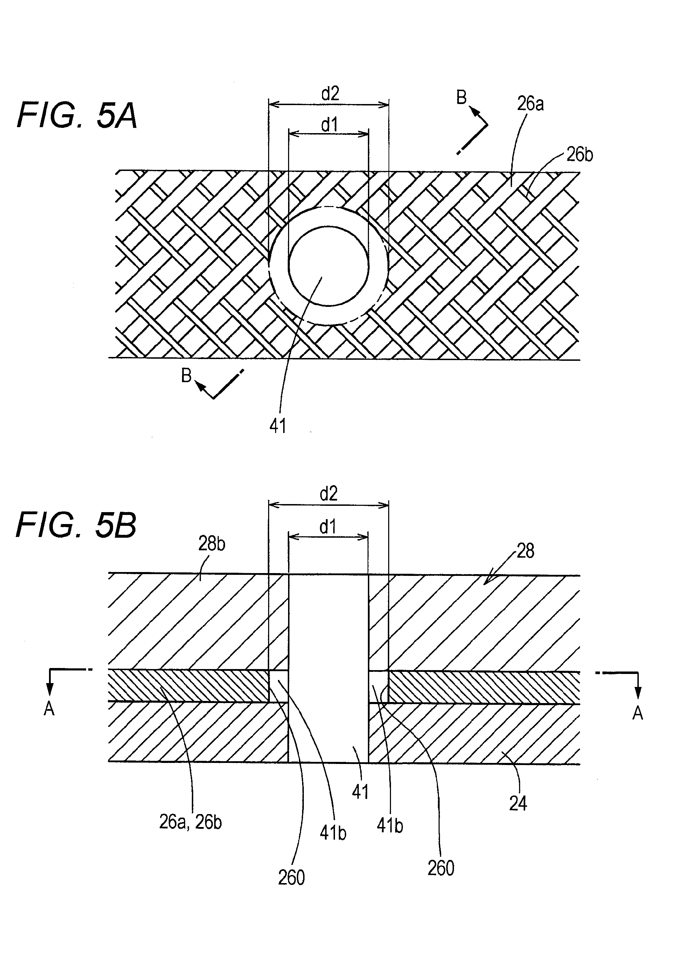

   Figs. 5A and 5B illustrate a second step for forming the side hole.
Figs. 6A and 6B

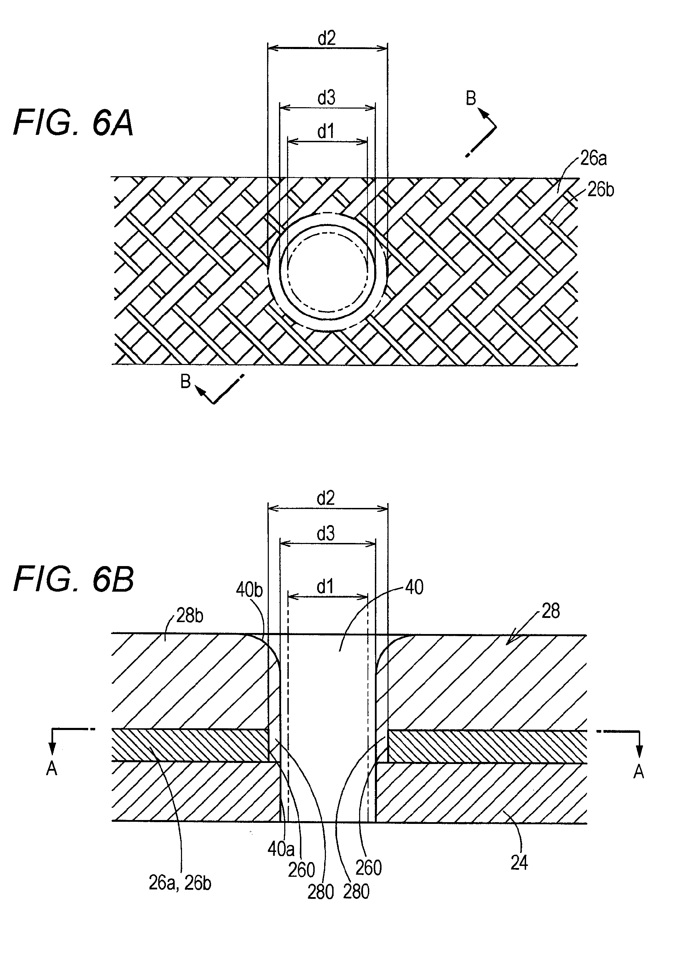

   Figs. 6A and 6B illustrate a completed side hole.
Fig. 7

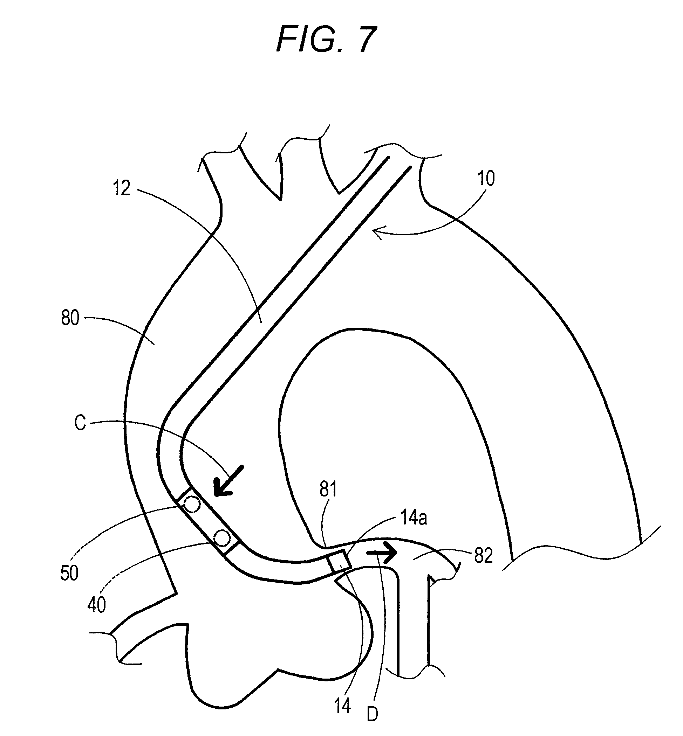

   Fig. 7 illustrates an explanatory view of an action of the embodiment.
Fig. 8

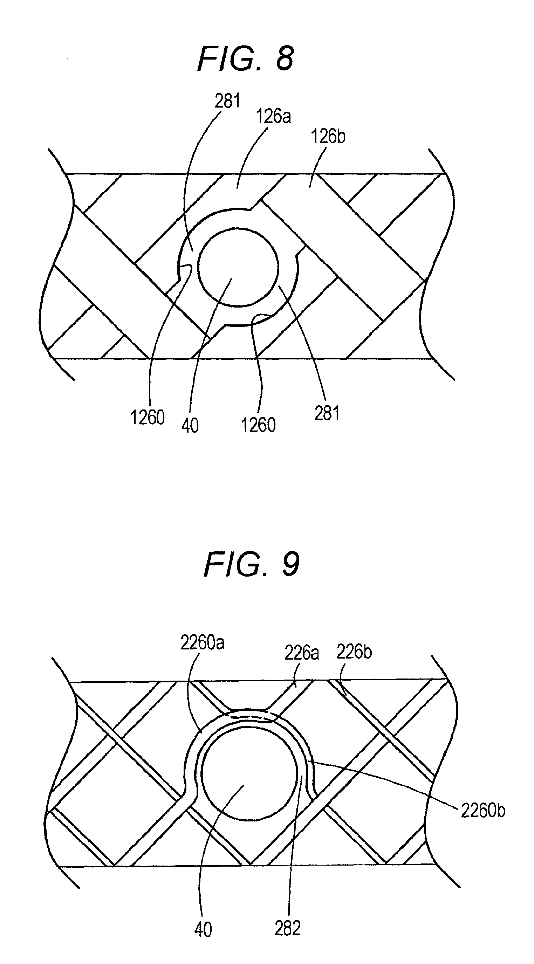

   Fig. 8 illustrates a second embodiment.
Fig. 9
   Fig. 9 illustrates a third embodiment.
Fig. 10

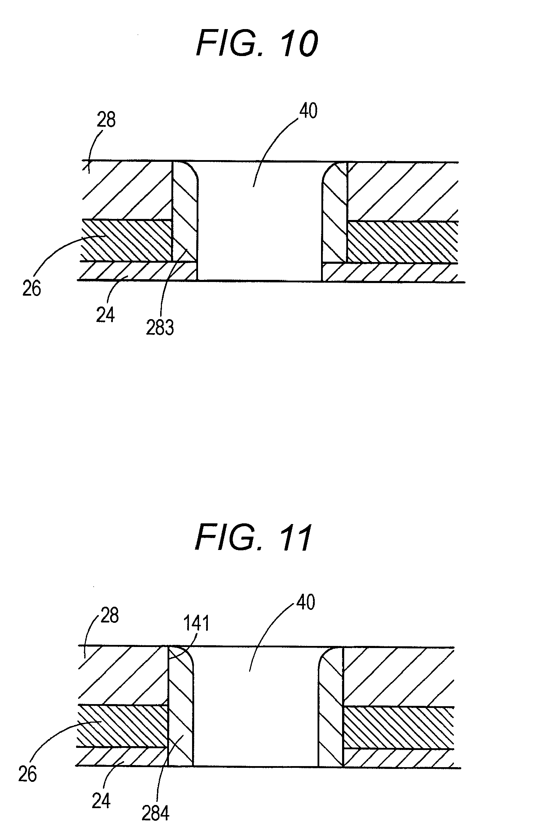

   Fig. 10 illustrates a fourth embodiment.
Fig. 11
   Fig. 11 illustrates a fifth embodiment.

### Description of Embodiments

<1> A medical tube according to a first aspect of the invention includes: an inner resin layer including a resinous tubular body having inside a lumen extending longitudinally; a braid member including at least one strand disposed around the outer periphery of the inner resin layer; an outer resin layer covering an outer periphery of the braid member; and at least one side hole passing through an outer resin layer and the inner resin layer to communicate with the lumen, the strand of the braid member provided in a vicinity of the side hole being covered with a resinous protective resin portion.
<2>A medical tube according to a second aspect of the invention is the medical tube according to the first aspect, wherein the strand of the braid member provided in the vicinity of the side hole is disposed at a distance from the inner peripheral surface of the side hole.
<3>A medical tube according to a third aspect of the invention is the medical tube according to the first aspect, wherein the strand of the braid member provided in the vicinity of the side hole is disposed at a distance from the inner peripheral surface of the side hole by having a different shape from the shape of the strand of the braid member provided in a portion other than the vicinity of the side hole.
<4>A medical tube according to a fourth aspect of the invention is the medical tube according to the first aspect, wherein the protective resin portion is configured by the outer resin layer extending to a side of the inner resin layer.
<5> A medial tube according to a fifth aspect of the invention is the medical tube according to the first aspect, wherein a distance between adjacent portions of the strand provided in a portion in which the side hole passes through the braid member is smaller than the maximum distance of an opening of the side hole.
<6>A catheter according to a sixth aspect of the invention is a catheter of the first aspect using the medical tube as a tubular catheter body.
<1> In the medical tube according to the first aspect of the present invention, the protective resin portion covers the strand of the braid member. Thus, the strand of the braid member is not exposed on the inner peripheral surface of the side hole. Because of this structure, the strand is protected from adherence of thrombi inside the side hole, thus enabling prevention of diminishing of the side hole in opening area during an operation. Accordingly, bloodstreams are stably secured by the structure of the side hole throughout the operation.

In addition, adherence of thrombi inside the side hole is prevented, and such needs are hence obviated as to enlarge the opening area of the side hole or to increase the number of the side hole preliminarily in consideration of the adherence of thrombi. Accordingly, reduction in structural strength is prevented to an utmost possible degree at the portion to be provided with the side hole in the medical tube. In addition, even when a side hole is formed on the braid member, structural weakening of the medical tube is prevented to an utmost possible degree. This helps reduce constraints in forming the side hole, such as to choose a portion where the braid member does not exist to form the side hole therein.

Moreover, the protective resin portion covers the strand of the braid member in the vicinity of the side hole, which obviates unanticipated risks such as inflicting damage on the inside of vessels by the strand projecting out of the side hole even in case where the medical tube is bent.
<2> According to the second aspect of the invention, the effect of the first aspect is easily achieved with the disposition of the strand of the braid member such that the strand is separated from the inner peripheral surface of the side hole. This structure is easily achieved by solving and removing a portion of the strand of the braid member through, e.g., electrolytic polishing.
<3> According to the third aspect of the invention, the effect of the first aspect is easily achieved with the deformation of the strand of the braid member into a different shape from the shape of the strand provided in a portion other than the vicinity of the side hole, so that the deformed strand of the braid member is spaced from the inner peripheral surface of the side hole. The strand of the braid member is not disconnected in this structure. The portion provided with the side hole is hence prevented to an utmost possible degree from being structurally weakened.
<4> According to the fourth aspect of the invention, the configuration of the aspect is easily achieved, for the outer resin layer forms the protective resin portion.
<5> According to the fifth aspect of the invention, in the side hole passing portion of the braid member, the strand is kept from adherence of thrombi by the protective resin portion even in case where the spacing between adjacent portions of the strand is smaller than the maximum distance of the opening of the side hole, namely in case where the side hole structurally inevitably cuts off a portion of the strand. The fifth aspect therefore provides an advantageous medical tube.
<6>According to the sixth aspect of the invention, the construction of the first aspect is applied to a catheter, thereby providing a catheter capable of keeping thrombi from adhering to the side hole and with which bloodstreams are secured stably. Further, the strand of the braid member is not exposed inside the side hole. Hence, a safe catheter is provided, which is capable of preventing to an utmost possible degree unanticipated risks such as inflicting damage on the inside of vessels with an exposed strand in a situation like the catheter being bent.

The medical tube of an embodiment is described exemplifying a case in which a cardiac guiding catheter 10 illustrated in Figs. 1 to 7 is used. In the figures, elements that are relatively smaller than the other portions, such as strands 26a and 26b of a braid member 26 and side holes 40 and 50 to be described hereinafter, are illustrated in a little exaggerated manner in a dimensional relationship with other elements for the sake of understandability.

The guiding catheter 10 illustrated in Fig. 1 is a tubular medical instrument. The catheter 10 includes a flexible catheter body 12 (medical tube), a chip 14 fixed at a distal end portion of the catheter body 12, and a connector 16 fixed at a proximal end portion of the catheter body 12.

The distal portion of the catheter body 12 is provided with a curved portion 112. The curved portion 112 is provided for the tip portion of the catheter 10 to be engaged in a coronary artery ostium when the catheter 10 is inserted into a human body.

More specifically, the chip 14 is a cylindrical member forming a tip opening 14a of the catheter 10 and is made from a resin such as a polyurethane elastomer. The chip 14 has an axial length on the order of 3.0 mm.

As illustrated in Fig. 2, the catheter body 12 is a tubular member that has an outer diameter on the order of 1.5 to 3.0 mm. The catheter body 12 has an inner resin layer 24 inside. The inner resin layer 24 forms a lumen 18 for receiving a guide wire or another catheter. Polytetrafluoroethylene (PTFE) is used, but not limited thereto, as the resin material of the inner resin layer 24 in the present embodiment.

The braid member 26 is disposed on a surface of the inner resin layer 24. As illustrated in Figs. 2 and 3, the braid member 26 is configured with a plurality of metal strands 26a and 26b braided into mesh (a net-like structure). In case of the present embodiment, eight by eight, i.e., 16 in total, metal strands 26a and 26b are braided alternately. As illustrated in Fig. 3, the strands 26a and 26b are coiled around the surface of the inner resin layer 24 at a pitch per wind of about 3.0 mm.

Strands 26a and 26b of different widths are used in the present embodiment, the strands being substantially rectangular in cross section. The strand 26a has a width of about 0.14 mm, and the strand 26b has a width of about 0.11 mm. The strands of the braid member 26 may be arranged in a net-like structure with the same kind of strands combined, besides the different kinds of strands as described above. The braid member also need not have a mesh-like structure and may have a coil-like structure formed of a single strand.

Further, the cross-sectional shape of the strands 26a and 26b is not particularly limited to the substantial rectangle and may take any other shape such as a round shape or a square.

The metal material of the strands 26a and 26b is also not particularly limited. For example, stainless steel (SUS 304 according to the JIS standard) or tungsten is used as the material. Stainless steel (SUS 304) is used in the present embodiment.

The surface of the braid member 26 is covered with an outer resin layer 28. The outer resin layer 28 includes four portions, i.e., a first outer resin layer 28a, a second outer resin layer 28b, a third outer resin layer 28c, and a fourth outer resin layer 28d, in the order from the distal side, and the hardness of the resin increases in this order. The resin material of the outer resin layer 28 is also not particularly limited. For example, polyamide, polyester, and polyurethane are used as the material. Polyamide is used in the present embodiment.

The first and second outer resin layers 28a and 28b have axial lengths of about 10.0 mm, respectively. The third outer resin layer 28c has a predetermined length that is longer than the lengths of the first outer resin layer 28a and of the second outer resin layer 28b. The remaining portion of the outer resin layer 28 serves as the fourth outer resin layer 28d.

As illustrated in Figs. 1 and 7, two side holes 40 and 50 are provided in the portion of the second outer resin layer 28b. The side holes 40 and 50 pass through the braid member 26 and the inner resin layer 24 from the outer surface of the second outer resin layer 28b to the lumen 18. The side holes 40 and 50 are round and are on the order of 0.7 to 1.0 mm in diameter (the maximum distance of the opening). The diameter is preferably set on the order of 0.8 to 0.9 mm in accordance with the outer diameter of the catheter body 12.

The number and diameter of the side holes are not limited to the above and may be set appropriately as required.

As apparent from the above-mentioned pitches and widths of the strands 26a and 26b of the braid member 26, as well as the diameter dimension of the side holes 40 and 50, the distance La between adjacent strands 26a and the distance Lb between adjacent strands 26b (the distances are defined by an edge portion of a first strand to an edge portion of a second strand) illustrated in Fig. 3 are both smaller than the diameter of the side holes 40 and 50 (the maximum distance of the µopening). For this reason, the side holes 40 and 50 are bored with at least portions of the strands 26a and 26b cut off. More specifically, some of the strands 26a and 26b are completely disconnected or portions of the strands 26a and 26b are cut out due to the formation of the side holes 40 and 50.

The side holes 40 and 50 are bored perpendicularly to the longitudinal axis of the catheter body 12 in a side surface of the curved portion 112 of the catheter body 12, more particularly, in a side surface at which the curved portion 112 forms a substantial plane. The positions at which the side holes 40 and 50 are bored are set on the proximal side at a distance of about 10.0 mm or more from the tip of the catheter 10. The positions are preferably at a distance on the order of 15.0 to 30.0 mm from the tip of the catheter 10. The positions at which the side holes are bored preferably fall within a less curved, substantially linear portion of the curved portion 112, such as the portion of the second outer resin layer 28b. This is because providing the side holes 40 and 50 in a greatly curved portion will cause a wide bend in the portion while the catheter 10 is in use, and the load incidental to the bend is prone to invite a kink (yielding or dent) in the catheter body 12.

Next, the structure of the side hole 40 is described with reference to Figs. 4A to 6B. Figs. 4A, 5A, and 6A illustrate top views of the portion having the side hole 40 formed therein with the outer resin layer 28 removed, while Figs. 4B, 5B, and 6B illustrate cross-sectional views of the portion of the catheter body 12, the portion having the side hole 40 formed therein. The side hole 50 basically has the same structure as that of the side hole 40, and thus the description thereof is not given.

In Figs. 6A and 6B, the inner diameter of the side hole 40 in a completed state is denoted by the reference numeral d3. The opening of the side hole 40 at the side of the outer resin layer 28 is enlarged with an arcuate taper portion 40b being provided. To avoid exposure of the respective endfaces 260 of the strands 26a and 26b of the braid member 26 at the inner peripheral surface 40a of the side hole 40, the endfaces 260 are covered with a portion of the resin constituting the outer resin layer 28 (the second outer resin layer 28b) to serve as a protective resin portion 280.

The method of forming the side hole 40 is described next with reference to Figs. 4A to 6B.

First, as illustrated in Figs. 4A and 4B, an initial hole 41 is formed from the side of the outer resin layer 28 (the second outer resin layer 28b) to the lumen 18. The inner diameter d1 of the initial hole 41 is set slightly smaller than the final inner diameter d3 of the side hole 40. For example, laser processing is used to bore the initial hole 41 in the catheter body 12.

In the state where the initial hole 41 is formed, the endfaces 261 of the strands 26a and 26b of the braid member 26 correspond to the inner peripheral surface 41a of the initial hole 41 and are exposed on the inner peripheral surface 41a.

Next, as illustrated in Figs. 5A and 5B, the end portions of the strands 26a and 26b of the braid member 26 are removed until the endfaces 260 (see Figs. 4B) of the strands 26a and 26b of the braid member 26 are recessed inward from the inner peripheral surface 41a of the initial hole 41. Such removal of the end portions of the strands 26a and 26b provides space 41b. The endfaces 261 of the braid member 26 recede to be endfaces 260. The diameter d2 of the circle defined by the endface 260 (shown with a dash line in Fig. 5A) and the inner diameter d1 of the initial hole 41 are set to a ratio d2/d1 on the order of 1.35 to 1.65.

The removal of the end portions of the strands 26a and 26b of the braid member 26 may also be effected through a mechanical method using a tool. Additionally, the removal may be achieved even more easily through a method, such as an electrolytic polishing or chemical polishing, of solving the metallic strands 26a and 26b.

Lastly, as illustrated in Figs. 6A and 6B, the outer resin layer 28 (the second outer resin layer 28b) is compressed into the space 41b formed by the removal of the braid member 26, such that the endfaces 260 of the braid member 26 not be exposed on the inner surface of the hole. The resin of the end portions of the outer resin layer 28 compressed serves as the protective resin portion 280 by covering the endfaces 260 of the braid member 26 as well as constituting a portion of the inner peripheral surface 40a of the side hole 40. The final inner diameter d3 of the diameter of the hole results from the compression, and an arcuate taper portion 40b is formed along the opening on the outer resin layer 28. As described above, in consideration of the need to compress the outer resin layer 28 into the space 41b, the final inner diameter d3 is set slightly larger than the inner diameter d1 of the initial hole 41.

The compression of the outer resin layer 28 is achieved with a tool (a punch) heated to a predetermined temperature by applying mechanical pressing force. The tool includes a cylindrical portion having such a diameter as to provide the final inner diameter d3 and an arcuate portion for forming the taper portion 40b at the opening on the outer resin layer 28.

Based on the above configuration, a description is hereafter given of the operation of the present embodiment exemplifying a case in which the guiding catheter 10 using the catheter body (medical tube) 12 is used in a cardiac operation.

Fig. 7 illustrates an example in which the catheter 10 is inserted from a radial artery of the wrist portion of a patient, through an aorta 80, to be in engagement with a left coronary artery ostium 81. In performing a cardiac treatment, a treatment catheter such as a balloon catheter or a guide wire (not illustrated) is passed inside the lumen 18 of the catheter 10 in this state. At this point, the distal end portion of the catheter 10 engages the left coronary artery ostium 81. For this reason, the distal end portion of the catheter 10 may block the left coronary artery ostium 81, leading to a condition deficient in the blood (so-called an ischemic condition) to flow from the aorta 80 into the left coronary artery 82.

As a preventive measure, the side holes 40 and 50 are provided in a side surface of the catheter 10. More specifically, as denoted by the arrows C and D in Fig. 7, blood in the aorta 80 flows from a lateral side of the catheter 10 through the side holes 40 and 50 into the lumen 18 to run out from the opening 14a provided at the distal end portion of the catheter 10. This assures blood influx from the aorta 80 into the left coronary artery 82.

Although the side holes 40 and 50 are bored at a side surface of the catheter body 12 to pass through the internal braid member 26, the protective resin portion 280 configured by the end portions of the outer resin layer 28 covers the endfaces 260 of the strands 26a and 26b of the braid member 26. Because of this structure, the strands 26a and 26b of the braid member 26 are not exposed on the inner peripheral surface 40a of the side hole 40. Thus, such a situation becomes avoidable that thrombi adhere to the strands 26a and 26b exposed in the side holes 40 and 50. Accordingly, shrinkage of the side holes 40 and 50 in opening area is avoided during the operation, and so stable blood flow is stably secured from the aorta 80 into the left coronary artery 82 during the operation.

In addition, the capability of preventing adherence of thrombi to the side holes 40 and 50 obviates the need to preliminarily enlarge the opening area of the side holes 40 and 50 or to increase the number of side holes in consideration of the adherence of thrombi. This can prevent, to an utmost possible degree, reduction in structural strength of the catheter body 12 due to lack or absence of the braid member 26 in the portion of the catheter body 12 having the side holes 40 and 50 formed therein. Also prevented to an utmost possible degree is lack or absence of the braid member due to formation of large or many side holes 40 and 50 on the braid member 26. This allows for reduction in design constraint in forming side holes, including, e.g., setting the positions of side holes 40 and 50 by choosing a portion having no braid member 26, and adjusting the spacing between the strands of the braid member.

Further, since the protective resin portion 280 of the outer resin layer 28 covers the endfaces 260 of the strands 26a and 26b of the braid member 26, even if the catheter body 12 is bent during an operation, the strands 26a and 26b do not project out of the side holes 40 and 50. Hence, unanticipated risks, such as damage being inflicted on the inside of vessels by strands, are also obviated.

The respective inner peripheral surfaces 40a of the side holes 40 and 50 have the taper portions 40b on the side of the outer resin layer 28. This eliminates contact of the corners of the openings of the side holes 40 and 50 on the inside of vessels in case where the catheter body 12 is bent during an operation. Thus, unanticipated risks such as damaging on the inside of vessels may further be eliminated.

In the foregoing embodiment, the strands 26a and 26b of the braid member 26 that are disposed between the outer resin layer 28 and the inner resin layer 24 are selectively removed by, e.g., electrolytic polishing. The resin of the outer resin layer 28 is compressed into the space thus formed between the outer resin layer 28 and the inner resin layer 24, so that the strands 26a and 26b of the braid member 26 are prevented from being exposed into the side hole 40. In this manner, the outer resin layer 28 is made use of, which obviates the necessity of an extra additional member for filling up the space between the outer resin layer 28 and the inner resin layer 24, allowing for prevention of exposure of the braid member 26 in the side hole 40 through a remarkably simple method.

Fig. 8 illustrates a second embodiment using electrolytic polishing. Of strands 126a and 126b of the braid member 26, electrolytic polishing is applied for removal of a strand 126a disposed in the vicinity of the side hole 40, such that this strand 126a is disposed apart from the inner peripheral surface of the side hole 40 and has a portion 1260 removed around the outer periphery of the side hole 40. Then, the resin of the outer resin layer is compressed into the space resulting from the removal of the strand 126a so as to form a protective resin portion 281. This allows for prevention of exposure of the strand 126a of the braid member 26 in the side hole 40.

Meanwhile, in a third embodiment illustrated in Fig. 9, of strands 226a and strands 226b of the braid member 26, a strand 226a and a strand 226b that are disposed in the vicinity of the side hole 40 are deformed and separated from the inner peripheral surface of the side hole 40 so as to have different shapes from the shapes of the strands 226a and 226b of the braid member 26 that are disposed in the portion other than the vicinity of the side hole 40. The strands 226a and 226b that are disposed in the vicinity of the side hole 40 have curved portions 2260a and 2260b, respectively, in such a manner as to surround the outer periphery of the side hole 40. Thus, the strands 226a and 226b are curved for spacing from the inner peripheral surface of the side hole 40, and a protective resin portion 282 is formed between the curved portions 2260a and 2260b and the side hole 40, which allows for prevention of the strands 226a and 226b of the braid member 26 from being exposed into the side hole 40. In this embodiment, since the strands 226a and 226b themselves are deformed, cutout or disconnection of strands need not be performed. The medical tube is therefore prevented to an utmost possible degree from being weakened structurally due to absence of the braid member.

Fig. 10 illustrates a fourth embodiment, which is still another embodiment of the invention. In this embodiment, removal is effected from the outside of the outer resin layer 28 up to the strands 26a and 26b of the braid member 26 with the inner resin layer 24 left. Then, in this embodiment, a protective resin portion 283 constructed by another tubular resin member is fitted in the removed portion; the braid member 26 is thereby prevented from being exposed into the side hole 40.

In a fifth embodiment illustrated in Fig. 11, a little large initial hole 141 is made in advance, and a protective resin portion 284 constructed of another tubular resin member is fitted in the initial hole 141 to provide the illustrated arrangement.

The resin forming the protective resin portions 283 and 284 may be the same as or may be different from that of the outer resin layer 28. Choice of a different resin enables use of a resin that is comparatively resistant to adherence of thrombi like coated resins and polytetrafluoroethylene (PTFE) to name a few. In this case, further advantageous effects are observed, for example, possibility of the use of, e.g., a hard resin that hardly permits the strands 26a and 26b of the braid member 26 to be exposed therethrough.

In the foregoing embodiments, the openings of the side holes 40 and 50 are substantially circular. The shape of the openings however is not particularly limited. The openings may take various shapes such as elliptical, oval, quadrangular, polygonal, including hexagonal, shapes.

Further, in the foregoing embodiments, a catheter body 12 with a three-layer structure comprising an outer resin layer 28, an inner resin layer 24, and a braid member 26 is illustrated. The catheter body 12 however may be structured such that another intermediate resin layer covering the braid member 26 is provided. In this case, the protective resin portion is to be constituted not only by the outer resin layer but also by the intermediate resin layer.

Further, in the foregoing embodiments, a medical tube of an embodiment of the invention is applied to a guiding catheter; however, the medical tubes of the embodiments of the present invention are not limited to guiding catheters and may be applied to various tubular medical instruments in addition to other kinds of catheters such as angiographic catheters.

While the invention has been shown and described in detail, the foregoing description is in all aspects illustrative and not restrictive. It is therefore understood that numerous modifications and variations can be devised without departing from the spirit and scope of the invention.

## Claims

1. A medical tube comprising:
an inner resin layer including a resinous tubular body having a lumen extending longitudinally;
a braid member including at least one strand disposed around an outer periphery of the inner resin layer;
an outer resin layer covering an outer periphery of the braid member; and
at least one side hole passing through the outer resin layer and the inner resin layer to communicate with the lumen,
the strand of the braid member provided in a vicinity of the side hole being covered with a resinous protective resin portion.

2. The medical tube according to claim 1, wherein the strand of the braid member provided in the vicinity of the side hole is disposed at a distance from the inner peripheral surface of the side hole.

3. The medical tube according to claim 1, wherein the strand of the braid member provided in the vicinity of the side hole is disposed at a distance from the inner peripheral surface of the side hole by having a different shape from the shape of the strand of the braid member provided in a portion other than the vicinity of the side hole.

4. The medical tube according to any of claims 1 to 3, wherein the protective resin portion is configured by the outer resin layer extending to a side of the inner resin layer.

5. The medical tube according to any of claims 1 to 4, wherein a distance between adjacent portions of the strand provided in a portion in which the side hole passes through the braid member is smaller than the maximum distance of an opening of the side hole.

6. A catheter using the medical tube of any of claims 1 to 5 as a tubular catheter body.
